# EUROPÄISCHE PATENTANMELDUNG

(11) **EP 4 640 197 A1**
(43) Veröffentlichungstag der Anmeldung: **29.10.2025**
(21) Anmeldenummer: 24171753.7
(22) Anmeldetag: 22.04.2024
(51) Int. Cl.: A61G 7/05, A61G 5/10, A61M 5/14

(54) **VORRICHTUNG ZUR ANORDNUNG AN EINEM PFLEGEHILFSMITTEL MIT EINEM GRUNDELEMENT UND EINEM ERWEITERUNGSELEMENT**

(71) Anmelder: Tobii Dynavox AB, 115 50 Stockholm (SE)
(72) Erfinder: Rabasse, Kenneth, 34414 Warburg (DE); Sprunck, Annemarie, 34266 Niestetal (DE); Mackerodt, Tim, 34121 Kassel (DE)
(74) Vertreter: Hannke Bittner & Partner mbB Kassel

(57) **Zusammenfassung**

Die Erfindung betrifft eine Vorrichtung (1) zur Anordnung an einem technischen Pflegehilfsmittel (2), und zeichnet sich dadurch aus, dass
a) sie ein erstes Grundelement (3) aufweist, welches an dem technischen Pflegehilfsmittel (2) lösbar aber unverlierbar befestigbar ist und dass,
b) an dem Grundelement (3) über ein Verbindungselement (4) wenigstens ein Erweiterungselement (5) befestigt ist, an welchem wenigstens eine Halterung (6) zur lösbaren aber unverlierbaren Anordnung einer medizin- oder pflegetechnischen Einrichtung (7) befestigt ist.

## Beschreibung

Die Erfindung betrifft eine Vorrichtung zur Anordnung an einem technischen Pflegehilfsmittel nach dem Oberbegriff des Patentanspruchs 1. Dabei sollen unter technischen Pflegehilfsmittel insbesondere Pflegebetten, Beistelltische für Pflegebetten, Rollstühle und dergleichen verstanden werden. Derartige technische Pflegehilfsmittel kommen insbesondere in der häuslichen Pflege aber auch in der stationären und ambulanten Pflege in Pflege- und Altenheimen zum Einsatz. Die Erfindung soll sich aber nicht auf diesen Bereich beschränken. Vielmehr kann sie auch bei der medizinischen Versorgung von Patienten in Krankenhäusern zum Einsatz kommen.

Im Rahmen der Pflege beziehungsweise der medizinischen Behandlung eines Patienten kommen gegebenenfalls eine Vielzahl von Pflegegerätschaften und medizinischen Geräten sowie auch Kommunikationsmittel zum Einsatz, die im Bereich des technischen Pflegemittels positioniert werden müssen, um sie für den Patienten und für das Pflegepersonal zugänglich zu machen. Dies können zum Beispiel Infusionsständer sein, aber auch Beatmungsgeräte sowie Luftbefeuchtungsgeräte dafür, Infusionspumpen, Blutdruckmessgeräte, EKG-Geräte, Displays zur Kommunikation von kommunikationsbeeinträchtigten Patienten mit ihrer Umgebung und dergleichen mehr. Das grundsätzliche Problem hierbei ist der begrenzte Platzverfügbarkeit beziehungsweise das begrenzte Platzangebot um das technische Pflegehilfsmittel herum, um die jeweilig benötigten Gerätschaften um den Patienten herum zu positionieren.

Laut der Deutschen Gesetzlichen Unfallversicherung (DGUV) gab es im Jahr 2020 allein in Deutschland insgesamt 23.067 meldepflichtige Arbeitsunfälle in der stationären und ambulanten Pflege. Dies entspricht einem Anstieg im Vergleich zu den Vorjahren. Die Zahl der Arbeitsunfälle in der Pflegebranche ist besorgniserregend hoch, und die Sicherheit und Gesundheit der Beschäftigten in diesem Bereich sind ein wichtiges Anliegen für die Arbeitsschutzbehörden und die Arbeitgeber. Einem Teil dieser Unfälle liegt die Ursache zugrunde, dass im Umfeld des technischen Pflegehilfsmittels auf dem Fußboden eine Vielzahl von Gerätschaften positioniert sind, die zur Pflege beziehungsweise Behandlung des Patienten notwendig sind, aber das Platzangebot um den Patienten derart beschränken, dass diese für das Pflege- beziehungsweise Behandlungspersonals Stolperfallen bilden, die zu Unfällen und damit zu Verletzungen des Pflege- beziehungsweise Behandlungspersonals führen können. Unfallquellen können dabei beispielsweise Kabel für die elektrische Versorgung der notwendigen Gerätschaften oder auch Infusionsständer, insbesondere rollbare, oder dergleichen mehr sein.

Es ist daher Aufgabe der Erfindung, eine Vorrichtung nach dem Oberbegriff des Patentanspruchs 1 zur Verfügung zu stellen, mit welcher die Gefahr potenzieller Unfälle in der häuslichen sowie stationären und ambulanten Pflege aber auch bei der medizinischen Versorgung von Patienten in Krankenhäusern zu reduzieren.

Gelöst wird diese Aufgabe durch eine Vorrichtung mit allen Merkmalen des Patentanspruchs 1. Vorteilhafte Ausgestaltungen der Erfindung finden sich in den Unteransprüchen.

Die erfindungsgemäße Vorrichtung zur Anordnung an einem technischen Pflegehilfsmittel zeichnet sich dadurch aus, dass
a) sie ein erstes Grundelement aufweist, welches an dem technischen Pflegehilfsmittel lösbar aber unverlierbar befestigbar ist und dass,
b) an dem Grundelement über ein Verbindungselement wenigstens ein Erweiterungselement befestigt ist, an welchem wenigstens eine Halterung zur lösbaren aber unverlierbaren Anordnung einer medizin- oder pflegetechnischen Einrichtung befestigt ist.

Durch die Erfindung wird eine direkte Anbindung der für die Pflege beziehungsweise Behandlung notwendigen Gerätschaften ermöglicht, sodass der Fußboden um das technische Pflegehilfsmittel von diesen Gerätschaften befreit wird und diese Gerätschaften keine Unfallgefahr am Fußboden um das technische Pflegehilfsmittel herum mehr darstellen. Die Gefahr von Stolperfallen durch Kabel oder Schläuche wird deutlich reduziert, da nunmehr alles ordentlich am technischen Pflegehilfsmittel befestigt ist. Dies erhöht die Sicherheit für den Patienten sowie für pflegende Angehörige und Pflegekräfte.

Zudem wird die Mobilität des technischen Pflegehilfsmittels samt den Gerätschaften, die zur Pflege beziehungsweise Behandlung des Patienten notwendig sind, verbessert. Alle notwendigen Geräte sind direkt am technischen Pflegehilfsmittel befestigt und mit ihm bewegbar, sodass sie nicht separat zum technischen Pflegehilfsmittel bewegt werden müssen. Dies erleichtert zum einen die Reinigung des Raumes und zum anderen ermöglicht es, den Patienten und das technische Pflegehilfsmittel bei Bedarf in einfacher Weise samt allen notwendigen Gerätschaften in einen anderen Raum zu bringen. Dadurch, dass alle notwendigen Geräte direkt am technischen Pflegehilfsmittel befestigt sind, sind sie für das Pflege- und Behandlungspersonal somit immer auch schnell und einfach erreichbar. Dies kann im Notfall entscheidend sein und erleichtert die tägliche Pflege- und Behandlungsroutine.

Weiterhin vorteilhaft ist, dass die Arbeitshöhe der Geräte optimal an das technische Pflegehilfsmittel angepasst werden kann, wodurch eine ergonomische Arbeitsweise für das Pflege- und Behandlungspersonal ermöglicht ist und körperliche Belastungen für diese reduziert sind. Ferner kann das gesamte System auf die spezifischen Bedürfnisse des Patienten sowie des Pflege- und Behandlungspersonal zugeschnitten werden, was eine personalisierte Pflegeumgebung schafft. Zudem wirkt ein derartig integriertes System oft aufgeräumter und ästhetisch ansprechender als viele einzelne Geräte, die um das technische Pflegehilfsmittel herum platziert sind, was insbesondere im häuslichen Umfeld bei einer häuslichen Pflege von Patienten von großem Interesse ist.

Vorteilhafterweise wird dadurch auch die Pflege und Behandlung des Patienten beschleunigt, da das Pflege- und Behandlungspersonal nicht zwischen verschiedenen Geräten hin- und herwechseln muss, sondern alles in Reichweite haben. Da alle Geräte fest installiert und organisiert sind, verringert sich auch das Risiko von Fehlbedienungen durch Verwechslungen oder falsche Einstellungen.

Langfristig können auch Kosten gespart werden, da weniger separate für zusätzliche Möbel benötigt werden und eventuell auch weniger Reparaturen anfallen, weil die Geräte besser gegen Beschädigungen geschützt sind, da ein Herunterfallen beziehungsweise Abstürzen der Geräte durch die feste Montage an dem technischen Pflegehilfsmittel vermieden ist. Auch können moderne Systeme in einfacher Weise als zusätzliche Module integriert werden.

Nach einer ersten vorteilhaften Ausgestaltung der Erfindung ist an dem Grundelement ein Stabilisierungselement befestigt ist, mit dem das Grundelement zusätzlich an dem technischen Pflegehilfsmittel befestigbar ist. Hierdurch wird in einfacher Weise eine stabile Vorrichtung ermöglicht, insbesondere dann, wenn eine Vielzahl von Gerätschaften mit erhöhtem Gewicht über die erfindungsgemäße Vorrichtung an dem technischen Pflegehilfsmittel befestigt werden sollen.

In die gleiche Richtung zielt die Ausgestaltung der Erfindung, dass an dem wenigstens einen Erweiterungselement ein Abstützelement angeordnet ist, welches mit dem technischen Pflegehilfsmittel lösbar aber unverlierbar verbindbar ist. Auch hierdurch wird analog zu dem Stabilisierungselementes des Grundelementes ein höher Gesamtstabilität erreicht, da auch das Erweiterungselement durch das Abstützelement zusätzlich stabilisiert wird.

Zusätzlich kann an dem wenigstens einen Erweiterungselement ein weiteres Grundelement angeordnet sein, welches mit dem technischen Pflegehilfsmittel lösbar aber unverlierbar verbindbar ist. Auch diese Ausgestaltung der Erfindung dient ebenso einer höheren Stabilität der gesamten erfindungsgemäßen Vorrichtung, wie die Ausgestaltung, dass an dem weiteren Grundelement ein Stabilisierungselement befestigt ist, mit dem das weitere Grundelement zusätzlich an dem technischen Pflegehilfsmittel befestigbar ist.

Zur einfachen Verbindung der erfindungsgemäßen Vorrichtung mit dem technischen Pflegehilfsmittel ist ein Verbindungselement vorgesehen, mit welchem das Grundelement an dem technischen Pflegehilfsmittel lösbar aber unverlierbar befestigbar ist.

Nach einer weiteren vorteilhaften Ausgestaltung der Erfindung ist an der wenigsten einen Halterung als medizin- oder pflegetechnischen Einrichtung ein Infusionsständer, ein Tablett, eine Kommunikationseinrichtung, ein Beatmungsgerät, eine Ablageeinrichtung, ein Köcher, ein Beatmungsgerät, ein Luftbefeuchtungsgerät für ein Beatmungsgerät, eine Infusionspumpe ein EKG-Gerät, ein Blutdruckmessgerät oder dergleichen befestigt ist. Durch die Vielzahl der medizin- oder pflegetechnischen Einrichtungen, die an der erfindungsgemäßen Vorrichtung befestigbar ist, ist das Gesamtsystem in einem hohen Grad individualisierbar, und kann daher zum einen sehr exakt an die Bedürfnisse des Patienten und zum anderen an die des Pflegebeziehungsweise Behandlungspersonals angepasst werden.

Vorteilhafterweise ist an dem wenigstens einen Erweiterungselement wenigstens eine Halterung zur lösbaren aber unverlierbaren Anordnung einer sicherheits- oder ordnungstechnischen Einrichtung befestigt ist. Eine derartige Einrichtung kann beispielsweise ein Kabelkanal sein, mit dem die elektrischen Zuleitungen der elektrisch betriebenen notwendigen Gerätschaften organisiert beziehungsweise geordnet werden können. Zudem kann eine derartige Einrichtung als Mehrfachstecker ausgebildet sein, über den alle elektrischen Zuleitungen der elektrisch betriebenen notwendigen Gerätschaften mit elektrischer Energie versorgt werden können. In beiden hier dargestellte Fällen, kann die Kabelführung der elektrischen Zuleitungen der elektrisch betriebenen notwendigen Gerätschaften optimiert und dadurch potenzielle Unfallquellen reduziert werden.

In einer Ausgestaltung der Erfindung ist die erfindungsgemäße Vorrichtung bereits an dem technischen Pflegehilfsmittel befestigt ist.

Als technisches Pflegehilfsmittel ist beispielsweise ein Pflegebett, ein Rollstuhl, ein Beistelltisch beziehungsweise -schrank oder dergleichen vorgesehen.

In einer besonders vorteilhaften Ausgestaltung der Erfindung sind das erste Grundelement und/oder das weitere Grundelement und/oder das wenigstens eine Erweiterungselement als Profile, vorzugsweise mit rundem Querschnitt ausgebildet. Dabei können insbesondere die Grundprofile als gebogene Profile ausgebildet sein, an denen das wenigstens eine Erweiterungselement derart befestigt ist, dass es parallel zum Fußboden verläuft, auf dem das technische Pflegehilfsmittel positioniert ist. Derartige Profile eignen sich in besonders guter Weise, um einen modularen Aufbau einer erfindungsgemäßen Vorrichtung zu realisieren und gewährleisten einen hohen Grad der Erweiterbarkeit. Dabei kann die erfindungsgemäße Vorrichtung auch nachträglich noch erweitert werden und so an sich ändernde Bedürfnisse des Patienten und auch des Pflege- und Behandlungspersonals angepasst werden
Insbesondere bei der Verwendung eines Pflegebettes als technisches Pfleghilfsmittel ist es vorteilhaft, dass das Verbindungselement in eine Einsteckhülse eines Pflegebettes einsteckbar ist und zur Aufnahme der Grundelemente ausgebildet ist. Pflegebetten sind nämlich bereits mit derartigen Einsteckhülsen ausgestattet, da diese zur Aufnahme eines Patientenaufrichters beziehungsweise eines Bettgalgens vorinstalliert sind.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: ein erstes Ausführungsbeispiel einer erfindungsgemäßen Vorrichtung in einer perspektivischen Darstellung,
- Figur 2:: das Ausführungsbeispiel der Figur 1 an einem Pflegebett befestigt,
- Figur 3:: ein zweites Ausführungsbeispiel einer erfindungsgemäßen Vorrichtung in einer perspektivischen Darstellung,
- Figur 4:: ein drittes Ausführungsbeispiel einer erfindungsgemäßen Vorrichtung in einer perspektivischen Darstellung,
- Figur 5:: ein viertes Ausführungsbeispiel einer erfindungsgemäßen Vorrichtung in einer perspektivischen Darstellung und
- Figur 6:: ein fünftes Ausführungsbeispiel einer erfindungsgemäßen Vorrichtung in einer perspektivischen Darstellung.

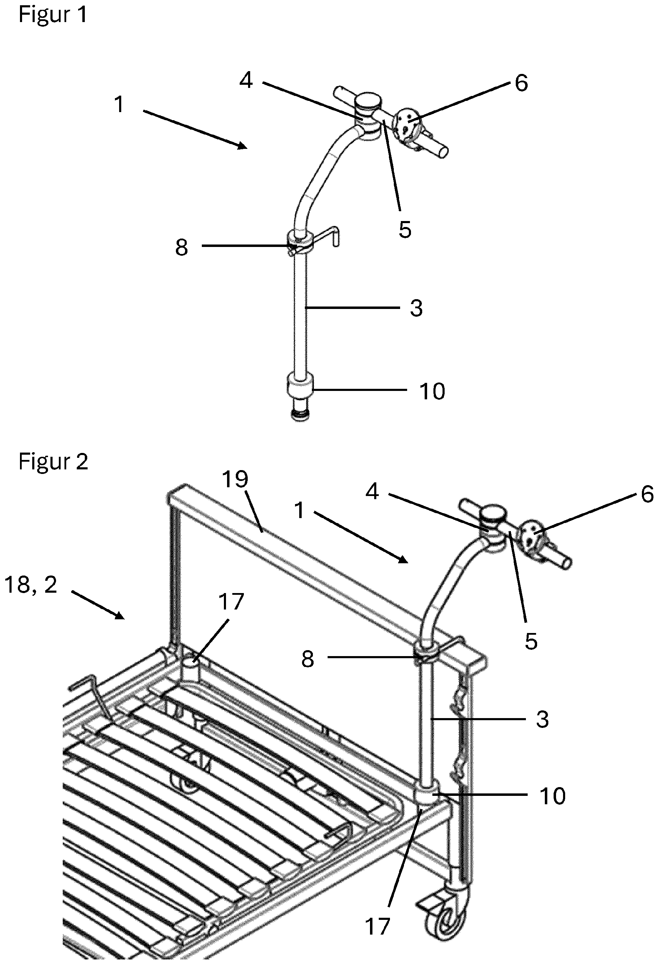

Die Figur 1 zeigt ein erstes Ausführungsbeispiel einer erfindungsgemäßen Vorrichtung 1 in einer perspektivischen Darstellung. Die erfindungsgemäßen Vorrichtung 1 besteht dabei im Wesentlichen aus einem Grundelement 3 und einem über ein Verbindungselement 4 verbundenes Erweiterungselement 5. Das Grundelement 3 weist dabei an seinem einen Ende ein Verbindungselement 10 auf, mit welchem es an einem technischen Pflegehilfsmittel 2 befestigbar ist. Wie aus der Darstellung der Figur 1 ersichtlich, handelt es sich bei dem Grundelement 3 um ein gebogenes Profil mit kreisrundem Querschnitt. Zwischen dem Verbindungselement 10 und dem Verbindungselement 4 ist an dem Grundelement 3 ein Stabilisierungselement 8 angeordnet, mit welchem es zusätzlich nochmals an den technischen Pflegehilfsmittel 2 befestigtbar ist, um somit die Stabilität der erfindungsgemäßen Vorrichtung 1 zu erhöhen. Das an dem Verbindungselement 4 angeordnete Erweiterungselement 5 ist ebenfalls als Profil mit kreisrundem Querschnitt ausgebildet. An diesem als Profil ausgebildeten Erweiterungselement 5 ist eine Halterung 6 angeordnet, an welcher ein pflege- oder medizintechnisches Gerät oder auch eine Kommunikationseinrichtung wie ein Display oder ein Tablet angeordnet werden können.

In der Darstellung der Figur 2 ist die erfindungsgemäße Vorrichtung 1 der Figur 1 bereits an einem als Pflegebett 18 ausgebildeten technischen Pflegehilfsmittel 2 angeordnet. Dabei ist das Grundelement 3 mit dem Verbindungselement 10 in einer Einstiegshülse 17 des Pflegebettes 18 eingesteckt. Derartige Einsteckhülsen 17 sind an einem Pflegebett 18 in der Regel vorinstalliert, da diese zur Anordnung eines Patientenaufrichters dienen. Des Weiteren ist das Grundelement 3 mit dem Stabilisierungselement 8 an einer Querleiste 19, welche sich am Ende des Kopfbereich des Pflegebettes 18 befindet, zusätzlich mit dem Pflegebett18 verbunden. Diese zusätzliche Verbindung dient der erhöhten Stabilität der erfindungsgemäßen Vorrichtung an dem Pflegebett 18.

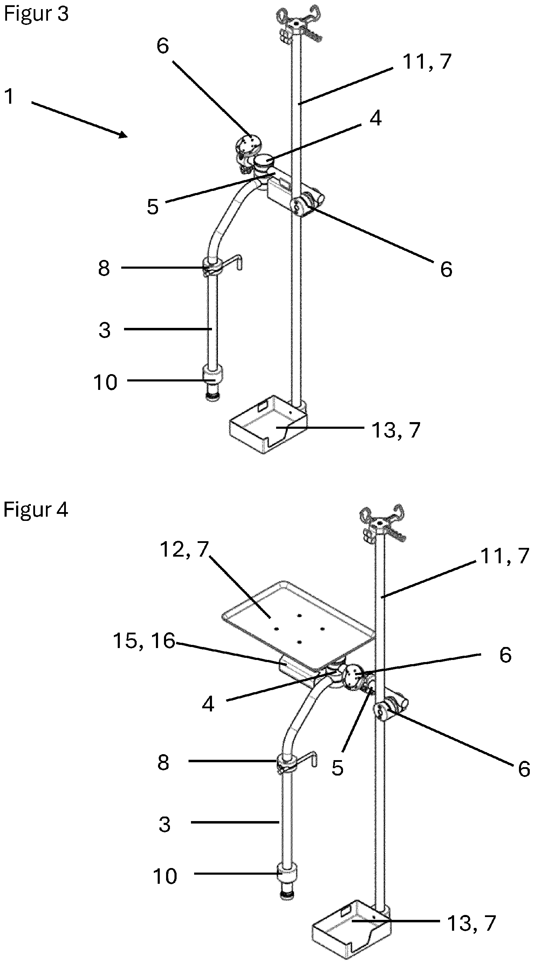

In der Figur 3 ist nunmehr ein zweites Ausführungsbeispiel einer erfindungsgemäßen Vorrichtung 1 in einer perspektivischen Darstellung gezeigt. Bei diesem Ausführungsbeispiel ist das Grundelement 3 und das Erweiterungselement 5 entsprechend dem Ausführungsbeispiel der Figuren 1 und 2 realisiert. Im Unterschied zu dem Ausführungsbeispiel der Figuren 1 und 2 sind an dem Erweiterungselement 5, welches wiederum über das Verbindungselement 4 mit dem Grundelement 3 verbunden ist, nunmehr zwei unterschiedliche Halterungen 6 angeordnet. Die linke Halterung 6 dient in diesem Ausführungsbeispiel wiederum zur Anordnung eines pflege- oder medizintechnischen Gerätes, wie beispielsweise eines Beatmungsgerätes, oder eines Kommunikationsgerätes, wie eines Displays oder eines Tablets.

An der im Gegensatz zum Ausführungsbeispiel der Figuren 1 und 2 zusätzlichen Halterung 6 ist in diesem Ausführungsbeispiel der erfindungsgemäßen Vorrichtung 1 eine Infusionsstange 11 angeordnet, an deren Ende eine Vorrichtung angeordnet ist, die zum einen einen Haken zur Anordnung eines Infusionsbeutels aufweist. Zum anderen sind noch weitere Elemente wie Schlauchführungen und ein Karabiner an dieser Vorrichtung angeordnet, um weitere Gegenstände daran anzuordnen beziehungsweise Schläuche, beispielsweise eines Beatmungsgerätes daran zu befestigen beziehungsweise zu führen. An dem dieser Vorrichtung gegenüberliegenden Ende der Iinfusionsstange11 ist eine Aufnahmeeinrichtung 13 angeordnet, auf welcher beispielsweise ein Luftbefeuchtungsgerät zur Verbindung mit einem Beatmungsgerät angeordnet beziehungsweise abgestellt beziehungsweise eingehängt werden kann.

Grundsätzlich wird bei dem Vergleich der beiden Ausführungsbeispiele der Figuren 1 bis 3 bereits jetzt deutlich, dass die erfindungsgemäße Vorrichtung 1 einen modularen Aufbau ermöglicht und individuell erweiterbar ist, sodass sie an die individuellen Bedürfnisse des Patienten beziehungsweise des Pflege- und/oder Behandlungspersonals angepasst werden kann. Durch die modulare Erweiterbarkeit der erfindungsgemäßen Vorrichtung 1 wird auch deutlich, dass das Grundelement 3 beziehungsweise das als dieses ausgebildete Profil eine höhere Belastung aushalten muss, je mehr Geräte beziehungsweise Vorrichtungen daran angeordnet sind. Insofern wird die Notwendigkeit der Verwendung des Stabilisierungselementes 8 nochmals verdeutlicht, wenn mehrere funktionale Geräte oder Vorrichtungen daran angeordnet sind. Durch die Verwendung des Stabilisierungselementes 8 erhält die gesamte erfindungsgemäße Vorrichtung 1 eine deutlich höhere Stabilität an dem technischen Pflegehilfsmittel 2 beziehungsweise dem Pflegebett 18.

Das in der Figur 4 dargestellte dritte Ausführungsbeispiel einer erfindungsgemäßen Vorrichtung 1 entspricht im Wesentlichen dem des zweiten Ausführungsbeispiels der Figur 3, wobei an dem als Profil mit kreisrunden Querschnitt ausgebildeten Erweiterungselement 5 nunmehr noch gegenüberliegend der Infusionsstange 11 über eine hier nicht erkennbare weitere Halterung 6 ein Tablett zu 12 zur Ablage verschiedene Gegenstände, insbesondere für das Pflege- und Behandlungspersonal, sowie eine als Kabelführung 16 ausgebildete sicherheits- oder ordnungstechnischen Einrichtung 15 angeordnet sind. Durch die Anordnung dieses Tabletts 12 und der Kabelführung 16 wird nochmals in eindrücklicher Weise verdeutlicht, dass durch die erfindungsgemäße Vorrichtung 1 in einfacher Weise eine modulare Erweiterung, insbesondere zur individuellen Anpassung an die Bedürfnisse des Patienten beziehungsweise des Pflege- und Behandlungspersonals möglich ist.

Durch die Verwendung einer Kabelführung 16 als sicherheits- oder ordnungstechnische Einrichtung 15 ist es möglich, elektrische Zuleitungen für medizintechnische oder kommunikationstechnische Geräte zu ordnen und zu organisieren und somit wahllos umher liegende Kabel und somit Unfallrisiken zu vermeiden. Zudem kann an der erfindungsgemäßen Vorrichtung 1 als weiteres Element ein Mehrfachstecker angeordnet werden, sodass alle elektrischen Geräte über diesen Mehrfachstecker mit elektrischer Energie versorgt werden können. Somit muss nur eine elektrische Leitung von dem technischen Pflegehilfsmittel2 beziehungsweise Pflegebett 18 weggeführt werden, um alle elektrischen Geräte, die mit der erfindungsgemäßen Vorrichtung verbunden sind mit elektrischer Energie zu versorgen.

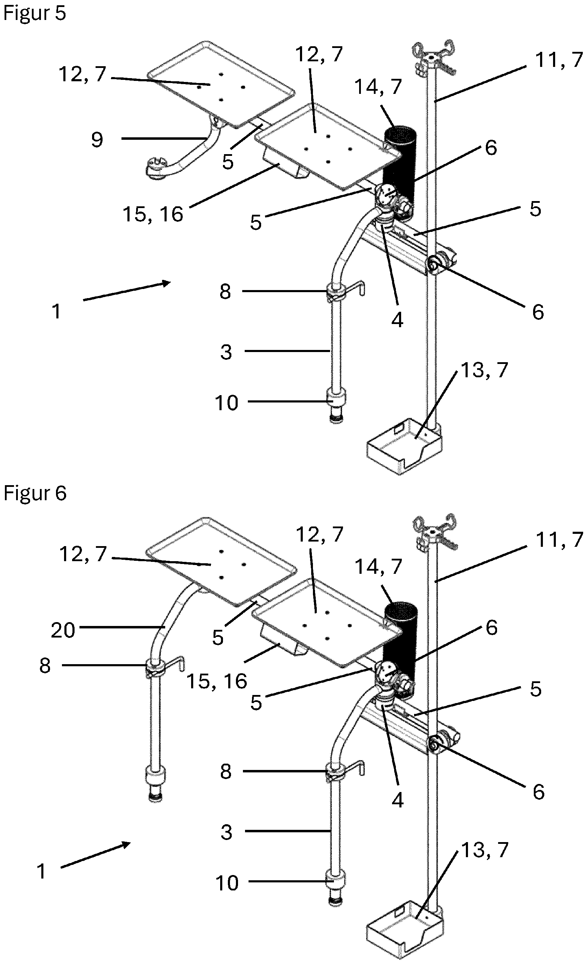

In der Figur 5 ist nun mehr ein viertes Ausführungsbeispiel einer erfindungsgemäßen Vorrichtung 1 dargestellt, bei welchem an dem Grundelement 3 nunmehr 2 Erweiterungselemente 5 angeordnet sind. Die an dem rechten Erweiterungselement 5 angeordnete Infusionsstange 11 entspricht dabei im Wesentlichen dem der Ausführungsbeispiele der Figuren 3 und 4.

An dem nun zusätzlich angeordneten weiteren Erweiterungselement 5 sind nunmehr zwei Tablets 12 sowie ein Köcher 14 zur Aufnahme verschiedener Gegenstände und eine Halterung 6 für die Aufnahme eines bereits in den vorherigen Ausführungsbeispiel beschriebenen weiteren Geräte angeordnet. Zusätzlich ist an dem weiteren Erweiterungselement 5 einen Abstützelement 9 angeordnet, welches sich an einer Querleiste 19 eines Pflegebettes 18 abstützen kann und zur Stabilitätserhöhung der erfindungsgemäßen Vorrichtung 1 dient. Auch enthält dieses Ausführungsbeispiel wiederum eine als Kabelführung 16 ausgebildete sicherheits- und ordnungstechnische Einrichtung 15 auf.

Das nunmehr in Figur 6 dargestellte weitere Ausführungsbeispiel einer erfindungsgemäßen Vorrichtung 1 entspricht im Wesentlichen dem des Ausführungsbeispiels der Figur 5. Im Gegensatz zu dem Ausführungsbeispiel der Figur 5 ist hierbei aber an dem weiteren Erweiterungselement 5 kein Abstützelement 9 angeordnet, sondern ein weiteres Grundelement 20, welches zur Verbindung der erfindungsgemäßen Vorrichtung 1 über ein weiteres Verbindungselement 10 mit einer weiteren Einsteckhülse eines Pflegebettes 18 ausgebildet ist. Auch weist dieses Grundelement 20 wiederum ein Stabilisierungselement 8 auf mit welchem das Grundelement 20 an einer Querleiste 19 im Kopfbereich eines Pflegebettes 18 zusätzlich befestigt werden kann, was nochmals die Stabilität der gesamten erfindungsgemäßen Vorrichtung 1 erhöht.

Grundsätzlich wird durch die erfindungsgemäßen Vorrichtung 1 der dargestellten Ausführungsbeispiele in eindrücklicher Weise verdeutlicht, dass die erfindungsgemäßen Vorrichtung 1 modular individuell erweiterbar beziehungsweise anpassbar ist und eine sichere Umgebung für das Pflege- und Behandlungspersonal sowie auch den Patienten ermöglicht.

### Bezugszeichenliste

- 1: Vorrichtung
- 2: technisches Pflegehilfsmittel
- 3: Grundelement
- 4: Verbindungselement
- 5: Erweiterungselement
- 6: Halterung
- 7: medizin- oder pflegetechnische Einrichtung
- 8: Stabilisierungselement
- 9: Abstützelement
- 10: Verbindungselement
- 11: Infusionsständer
- 12: Tablett
- 13: Aufnahmeeinrichtung
- 14: Köcher
- 15: sicherheits- oder ordnungstechnische Einrichtung
- 16: Kabelführung
- 17: Einsteckhülse
- 18: Pflegebett
- 19: Querleiste
- 20: Grundelement

## Patentansprüche

1. Vorrichtung (1) zur Anordnung an einem technischen Pflegehilfsmittel (2),
**dadurch gekennzeichnet, dass**
a) sie ein erstes Grundelement (3) aufweist, welches an dem technischen Pflegehilfsmittel (2) lösbar aber unverlierbar befestigbar ist und dass,
b) an dem Grundelement (3) über ein Verbindungselement (4) wenigstens ein Erweiterungselement (5) befestigt ist, an welchem wenigstens eine Halterung (6) zur lösbaren aber unverlierbaren Anordnung einer medizin- oder pflegetechnischen Einrichtung (7) befestigt ist.

2. Vorrichtung nach Anspruch 1, **dadurch gekennzeichnet, dass** an dem Grundelement (3) ein Stabilisierungselement (8) befestigt ist, mit dem das Grundelement (3) zusätzlich an dem technischen Pflegehilfsmittel (2) befestigbar ist.

3. Vorrichtung nach Anspruch 1 oder 2, **dadurch gekennzeichnet, dass** an dem wenigstens einen Erweiterungselement (5) ein Abstützelement (9) angeordnet ist, welches mit dem technischen Pflegehilfsmittel (2) lösbar aber unverlierbar verbindbar ist.

4. Vorrichtung nach Anspruch 1 oder 2, **dadurch gekennzeichnet**, an dem wenigstens einen Erweiterungselement (5) ein weiteres Grundelement (20) angeordnet ist, welches mit dem technischen Pflegehilfsmittel (2) lösbar aber unverlierbar verbindbar ist.

5. Vorrichtung nach Anspruch 1, **dadurch gekennzeichnet, dass** an dem weiteren Grundelement (20) ein Stabilisierungselement (8) befestigt ist, mit dem das weitere Grundelement (20) zusätzlich an dem technischen Pflegehilfsmittel (2) befestigbar ist.

6. Vorrichtung nach einem der vorhergehenden Ansprüche, **dadurch gekennzeichnet, dass** das Grundelement (3) über ein Verbindungselement (10) an dem technischen Pflegehilfsmittel (2) lösbar, aber unverlierbar befestigbar ist.

7. Vorrichtung nach einem der vorhergehenden Ansprüche, **dadurch gekennzeichnet, dass** an der wenigsten einen Halterung (6) als medizin- oder pflegetechnischen Einrichtung (7) ein Infusionsständer (11), ein Tablett (12), eine Kommunikationseinrichtung, ein Beatmungsgerät, eine Aufnahmeeinrichtung (13), ein Köcher (14), ein Beatmungsgerät, ein Luftbefeuchtungsgerät für ein Beatmungsgerät, eine Infusionspumpe ein EKG-Gerät, ein Blutdruckmessgerät oder dergleichen befestigt ist.

8. Vorrichtung nach einem der vorhergehenden Ansprüche, **dadurch gekennzeichnet, dass** an dem wenigstens einen Erweiterungselement (5) wenigstens eine Halterung zur lösbaren aber unverlierbaren Anordnung einer sicherheits- oder ordnungstechnischen Einrichtung (15) befestigt ist.

9. Vorrichtung nach Anspruch 8, **dadurch gekennzeichnet, dass** an Halterung zur lösbaren aber unverlierbaren Anordnung einer sicherheits- oder ordnungstechnischen Einrichtung (15) ein Kabelkanal, eine Kabelführung (16) oder dergleichen befestigt ist.

10. Vorrichtung nach einem der vorhergehenden Ansprüche, **dadurch gekennzeichnet, dass** sie an dem technischen Pflegehilfsmittel (2) befestigt ist.

11. Vorrichtung nach einem der vorhergehenden Ansprüche, **dadurch gekennzeichnet, dass** als technisches Pflegehilfsmittel (2) ein Pflegebett (18), ein Rollstuhl, ein Beistelltisch oder dergleichen vorgesehen ist.

12. Vorrichtung nach einem der vorhergehenden Ansprüche, **dadurch gekennzeichnet, dass** das Grundelement (3) und/oder das Grundelement (20) und/oder das wenigstens eine Erweiterungselement (5) als Profile, vorzugsweise mit rundem Querschnitt ausgebildet sind.

13. Vorrichtung nach einem der Ansprüche 6 bis 11, **dadurch gekennzeichnet, dass** das Verbindungselement (10) in eine Einsteckhülse (17) eines Pflegebettes (16) einsteckbar ist und zur Aufnahme der Grundelemente (3, 20) ausgebildet ist.
